# EUROPÄISCHE PATENTSCHRIFT

(11) **EP 1 759 626 B1**
(45) Veröffentlichungstag und Bekanntmachung des Hinweises auf die Patenterteilung: **22.05.2013**
(21) Anmeldenummer: 06016066.0
(22) Anmeldetag: 02.08.2006
(51) Int. Cl.: A61B 1/00

(54) **Endoskop**
Endoscope
Endoscope

(30) Priorität: 31.08.2005 DE 102005041454
(43) Veröffentlichungstag der Anmeldung: 07.03.2007
(73) Patentinhaber: Karl Storz GmbH & Co. KG, 78532 Tuttlingen (DE)
(72) Erfinder: Pilvisto, Tönis, 13912 Tallinn (EE); Wimmer, Viktor Josef, 83370 Seeon (DE)
(74) Vertreter: Hofmeister, Frank

(56) Entgegenhaltungen:
- EP-A1- 0 609 503
- EP-B1- 1 112 019
- DE-C1- 19 627 016

## Beschreibung

Die Erfindung betrifft ein Endoskop mit einem Schaft, in dem mindestens ein Arbeitskanal angeordnet ist und mit einem am distalseitigen Ende des Schaftes angeordneten Endoskopkopf, wobei der im Endoskopkopf angeordnete Teil des Arbeitskanals über einen Bowdenzug, der proximalseitig über eine Spannzange an einem Steuerungs-Handgriff gelagert ist, gegenüber dem zugehörigen Arbeitskanal verschwenkbar ist.

Um ein über den Arbeitskanal dem Operationsgebiet zugeführtes medizinisches Instrument gezielt dem Einsatzgebiet zuführen zu können ist es bekannt, den im Endoskopkopf angeordneten Teil des Arbeitskanals über einen Bowdenzug gegenüber dem zugehörigen Arbeitskanal verschwenkbar auszugestalten. Bei dem aus der EP 1 112 019 B1 bekannten Endoskop erfolgt die proximalseitige Lagerung des zum Verschwenken des Arbeitskanals dienenden Bowdenzugs über eine an einem Steuerungs-Handgriff gelagerte Spannzange. Über die Spannzange wird das Zugseil des Bowdenzugs klemmend ergriffen und entsprechend der Einstecktiefe in die Spannzange gespannt.

Diese bekannte Konstruktion zum Spannen des Bowdenzugs weist den Nachteil auf, dass das Zugseil so tief in die Spannzange eingeschoben werden kann, dass das Zugseil zu straff gespannt ist und ein Verschwenken des Arbeitskanals kaum noch möglich ist. Um diese Gefahr zu minimieren, muss der Arbeitskanal und somit auch das Zugseil des Bowdenzugs zunächst in eine vorbestimmte Ausgangsstellung überführt werden, damit nachfolgend eine ausreichende, aber nicht zu straffe Spannung des Zugseils mittels der Spannzange erfolgen kann. Dieses bekannte Spannverfahren ist einerseits umständlich und verhindert andererseits nicht eine Fehlspannung des Zugseils.

Davon ausgehend liegt der Erfindung die Aufgabe zugrunde, ein Endoskop der eingangs genannten Art so auszugestalten, dass bei einfachem Aufbau eine stets gleichmäßige Spannung des Bowdenzugs gewährleistet ist.

Die Lösung dieser Aufgabenstellung ist erfindungsgemäß dadurch gekennzeichnet, dass ein am Steuerungs-Handgriff gelagertes Andrückelement die Einstecktiefe eines Zugseils des Bowdenzugs in die Spannzange begrenzt, wobei an der Spannzange proximalseitig ein Konus ausgebildet ist, der mit einem Gegenkonus zusammenwirkt, der in einer auf die Spannzange aufschraubbaren Mutter ausgebildet ist und das Andrückelement über mindestens ein Federelement vorgespannt am Gegenkonus anliegt, wobei das mindestens eine Federelement in einer Sacklochbohrung der Mutter angeordnet ist.

Durch die erfindungsgemäße Limitierung der Einstecktiefe des Zugsseils des Bowdenzugs in die Spannzange ist es erstmalig möglich, eine stets gleichmäßige Einstecktiefe und somit auch gleichmäßige Spannung des Zugseils zu erhalten. Die Gefahr der Fehlspannung des Zugsseils bei der Montage des Bowdenzugs wird durch diese konstruktive Ausgestaltung aufgehoben.

Durch dieses Zusammenspiel von Konus und Gegenkonus wird eine sichere und verkanntungsfreie Lage der Spannzange in der Mutter gewährleistet, wobei im Konus der Spannzange mindestens ein in Längsrichtung der Spannzange verlaufender Klemmschlitz ausgebildet ist, wodurch das Zugseil beim Anlaufen des Spannzangenkonus gegen den Gegenkonus klemmend in der Spannzange fixiert wird.

Um zu gewährleisten, dass der Gegenkonus immer formschlüssig am Konus der Spannzange anliegt, wirkt das erfindungsgemäße Andrückelement über mindestens ein Federelement auf den Gegenkonus ein.

Weiterhin wird gemäß einer bevorzugten Ausführungsform der Erfindung vorgeschlagen, dass das Andrückelement als hutförmiges Bauteil ausgebildet ist, wobei die axiale Länge des Andrückelements der bevorzugten und maximalen Einstecktiefe des Zugseils in die Spannzange entspricht. Da das hutförmige Andrückelement federbelastet am Gegenkonus anliegt, der die Endlage der Spannzange in der Mutter definiert, ergibt sich eine immer gleiche und vorherbestimmbare Spannung des Zugseils, weil beim Aufschrauben der Mutter auf koaxial auf dem Zugseil angeordnete Spannzange das Zugseil proximalseitig nur der axialen Länge des Andrückelements entsprechend über die Spannzange hervorstehen kann. Durch das Andrückelement wird somit bei der Montage des Bowdenzugs am Steuerungs-Handgriff die Einstecktiefe des Zugseils beschränkt.

Weiterhin wird mit der Erfindung vorgeschlagen, dass sich das Federelement distalseitig an einem umlaufenden Kragen des hutförmigen Andrückelements und proximalseitig an einer Rückwand der als Hutmutter ausgebildeten Mutter abstützt.

Um sicherzustellen, dass das Zugseil des Bowdenzugs immer mittig zentriert im Andrückelement gelagert ist, wird mit der Erfindung vorgeschlagen, dass eine geschlossene Rückwand des hutförmig ausgebildeten Andrückelements nach außen weisend spitz zulaufend ausgebildet ist.

Schließlich wird mit der Erfindung vorgeschlagen, dass der Gegenkonus an einer in die Mutter einsetzbaren Hülse ausgebildet ist, wobei die Hülse, beispielsweise durch Verkleben, verdrehsicher in der Mutter fixierbar ist, um ein Verdrehen der mit dem Gegenkonus versehenen Hülse beim Aufschrauben der Mutter zu verhindern.

Um ein einfaches und verkantungsfreies Aufschrauben der Mutter auf die Spannzange zu ermöglichen, ist an der Hülse distalseitig ein gewindefreier Einstich ausgebildet. Dieser den Innendurchmesser der Hülse erweiternde Einstich ermöglicht eine Zentrierung der Spannzange in der Mutter.

Weitere Merkmale und Vorteile der Erfindung ergeben sich anhand der zugehörigen Zeichnung, in der ein Ausführungsbeispiel eins erfindungsgemäßen Endoskops nur beispielhaft dargestellt ist. In der Zeichnung zeigt:
- Fig. 1: eine perspektivische Seitenansicht des distalen Endes eines erfindungsgemäßen Endoskops;
- Fig. 2: eine Ansicht gemäß Fig. 1, jedoch mit abgenommener Endoskopkopfabdeckung;
- Fig. 3: eine perspektivische Seitenansicht des vom Endoskopschaft getrennten Endoskopkopfs
- Fig. 4: eine ausschnittweise Seitenansicht eines Steuerungshandgriffs für ein erfindungsgemäßes Endoskop;
- Fig. 5: eine vergrößerte und geschnittene Ansicht des Details V gemäß Fig. 4, jedoch den zusammengebauten Zustand darstellend;
- Fig. 6: eine Vorderansicht der Darstellung gemäß Fig. 2 und
- Fig. 7: eine Vorderansicht des Endoskopkopfes mit abgenommenem Endoskopkopf.

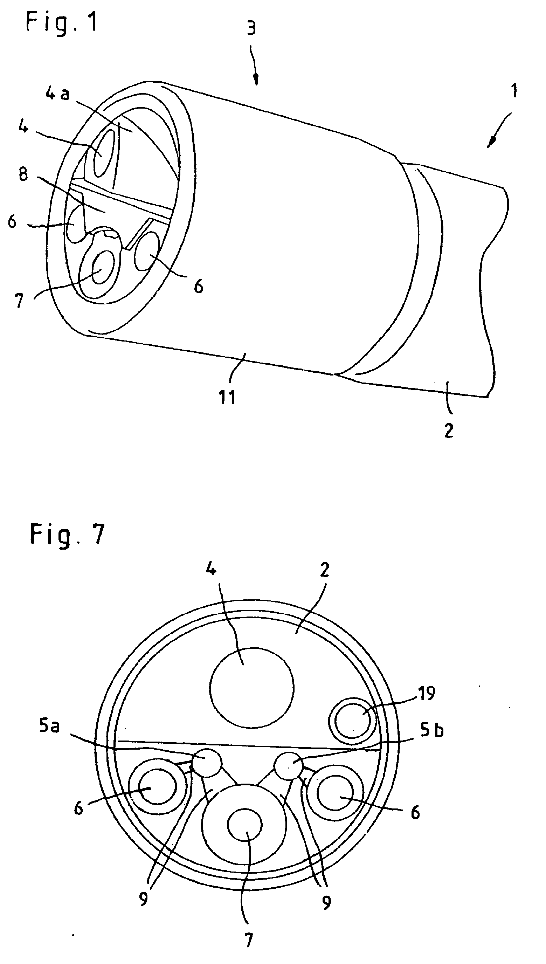

Abbildung Fig. 1 zeigt das distale Ende eines Endoskops 1 mit einem das distale Ende eines Endoskopschaftes 2 bildenden Endoskopkopf 3, welcher eine Verlängerung des Endoskopschaftes 2 bildet.

Im Inneren des Schaftes 2 sind, wie aus Fig. 7 ersichtlich, mehrere in Längsrichtung verlaufende Kanäle, nämlich ein Arbeitskanal 4, ein Spülkanal 5a, ein Luftkanal 5b sowie zwei Beleuchtungskanäle 6, angeordnet. Ferner erstreckt sich in Längsrichtung des gesamten Schaftes 2 die Optik 7 des Endoskops 1.

Um dafür zu sorgen, dass der mit dem Endoskop arbeitende Operateur jederzeit einen klaren Blick auf das Operationsgebiet hat, wird die aus dem Spülkanal 5a austretende Spülflüssigkeit über eine als Prallblech ausgebildete Führung 8 auf die Optik 7 sowie die in den Beleuchtungskanälen 6 angeordneten Lichtsysteme abgelenkt, wobei bei der dargestellten Ausführungsform die Spülflüssigkeit der Optik 7 sowie den Beleuchtungskanälen 6 über entsprechend angeordnete Zuführkanäle 9 zugeführt wird, wie dies insbesondere der Abbildung Fig. 7 zu entnehmen ist. Über den aus dem Luftkanal 5b austretenden Luftstrom werden nach Beendigung der Optikspülung die Spülflüssigkeitstropfen weggeblasen.

Zusätzlich zu diesem trocknen der Optik 7 dient der über den Luftkanal 5b zugeführte Luftstrom dazu, die Organe aufzublasen, um so das Endoskop 1 möglichst reibungsfrei in das Untersuchungsgebiet einschieben zu können.

Das Verbinden des Endoskopkopfs 3 mit dem Endoskopschaft 2 des Endoskops 1 erfolgt bei der dargestellten Ausführungsform über eine auf ein Außengewinde 10 des Endoskopkopfes 2 aufschraubbare Hülse 11, die gleichzeitig das Außengehäuse des Endoskopkopfs 3 bildet.

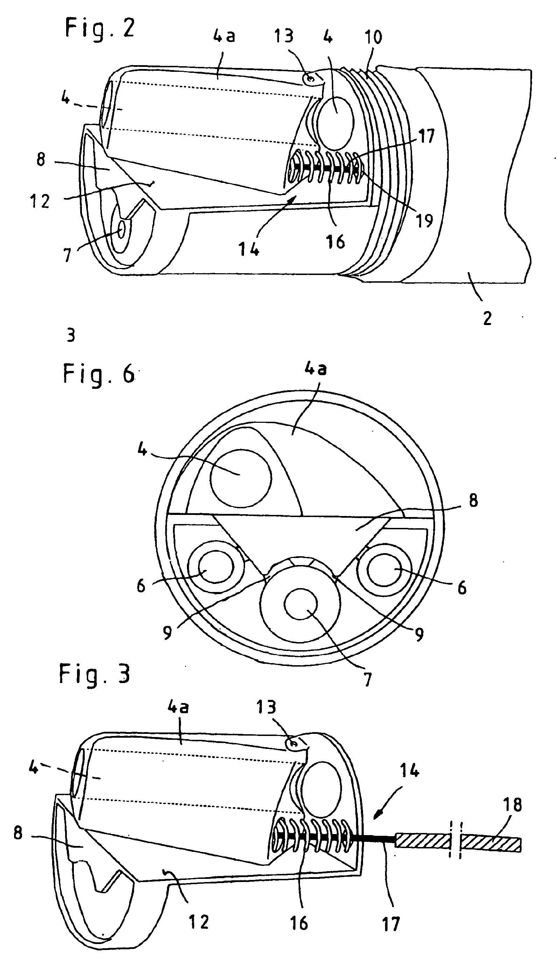

Fig. 2 zeigt schließlich eine Fig. 1 entsprechende Seitenansicht, jedoch bei abgenommener Hülse 11, so dass nunmehr der konstruktive innere Aufbau des Endoskopkopfs 3 erkennbar ist. Wie aus dieser Ansicht sowie der Abbildung Fig. 3 ersichtlich, ist der Endoskopkopf 3 als in Axialrichtung des Schafts 1 zweigeteiltes Gehäuse so aufgebaut, dass der Arbeitskanal 4 in dem einen Gehäuseteil und die Optik 7, die Beleuchtungskanäle 6 für die Lichtsysteme sowie die Spülkanale 5 in dem anderen Gehäuseteil angeordnet sind.

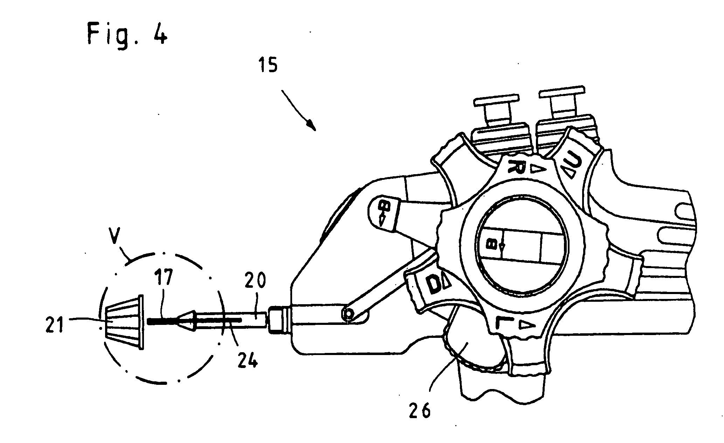

Die Verlängerung des Arbeitskanals 4 im Endoskopkopf 3 weist die Besonderheit auf, dass dieser Teil 4a in der Ebene des Aufnahmeblechs 12 verschwenkbar gegenüber dem zugehörigen Arbeitskanal 4 im Schaft 1 ausgebildet ist. Durch diese Verschwenkbarkeit des distalen Teils 4a des Arbeitskanals 4 ist es möglich, ein vom proximalen Ende des Endoskops 1 her in den Arbeitskanal 4 eingeführtes medizinisches Instrument gezielt dem Operationsgebiet zuzuführen. Das Verschwenken des distalen Teils 4a des Arbeitskanals 4 um eine Schwenkachse 13 erfolgt über einen im Schaft 1 gelagerten Bowdenzug 14, der über einen Steuerungs-Handgriff 15 am proximalen Ende des Endoskops 1 betätigbar ist, wie dies in Fig. 4 dargestellt ist.

Wie weiterhin aus den Abbildungen Fig. 2 und 3 ersichtlich, ist der verschwenkbare Teil 4a des Arbeitskanals 4 über ein Federelement 16 in eine Ausgangsstellung vorgespannt, um eine definierte Ausgangsstellung des Arbeitskanals 4 zu haben, von der ausgehend der verstellbare Teil 4a verschwenkbar ist. Durch diese Ausgangsstellung lässt sich ein Nullpunkt definieren, wobei die optische Achse eine durchaus geeignete Ausgangsstellung/Nullpunktstellung darstellt. Um auch bei Bewegungen diese Ausgangsstellung beizubehalten, ist es vorteilhaft eine Arretierung vorzusehen, über die der verschwenkbare Teil 4a des Arbeitskanals 4 in dieser Ausgangsstellung fixierbar ist.

Die Montage und Demontage des Bowdenzugs am verschwenkbaren Teil 4a des Arbeitskanals 4 sowie am Steuerungs-Handgriff 15 geschieht wie folgt:

Zu Beginn der Montage wird ein Zugseil 17 des Bowdenzugs 14 in einen Bowdenzugmantel 18 eingeschoben und anschließend der mit dem Zugseil 17 bestückte Bowdenzugmantel 18 vom distalen Ende des Endoskopkopfes 3 aus in einen im Schaft 1 ausgebildeten Bowdenzugkanal 19 eingeführt.

Nachfolgend wird der Endoskopkopf 3 über die Schraubhülse 11 am Endoskopschaft 2 festgelegt. Das Zugseil 17 wird soweit in den Bowdenzugmantel 18 hineingeschoben, bis das Zugseil 17 proximalseitig im Bereich des Steuerungs-Handgriffs 15 wieder aus dem Bowdenzugkanal 19 heraustritt. Das proximalseitige Festlegen des Zugseils 17 am Steuerungs-Handgriff 15 erfolgt über einen auf das Zugseil 17 aufsteckbare Spannzange 20, die durch das Aufschrauben einer Mutter 21 mit dem Zugseil 17 verklemmt wird. Über den Steuerungs-Handgriffs 15 lässt sich nunmehr der verschwenkbare Teil 4a des Arbeitskanals 4 verstellen.

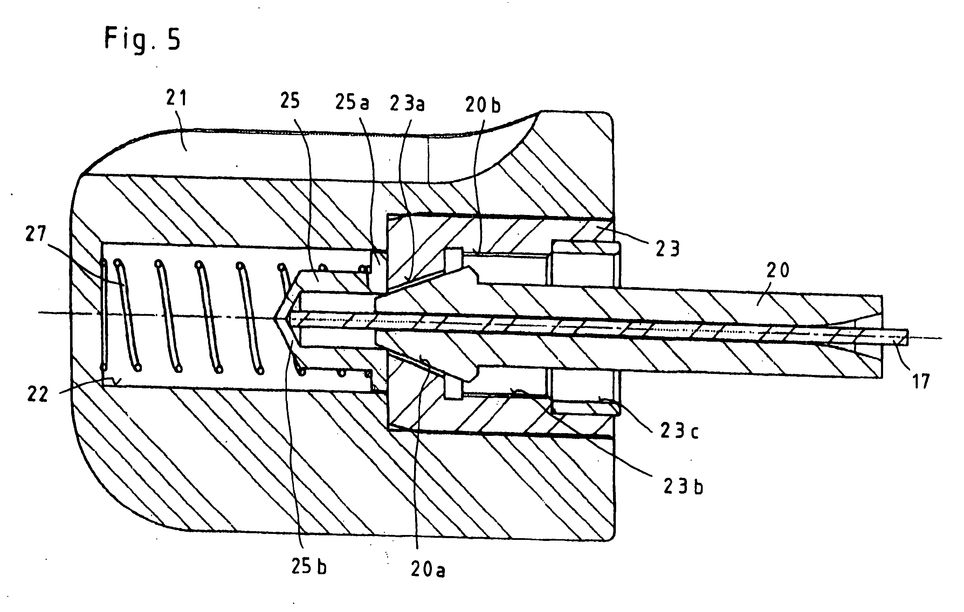

Der Aufbau der als Hutmutter ausgebildeten Mutter 21 und der Spannzange 20 sowie die Art und Weise der Spannung des Zugseils 17 ist der vergrößerten Detailansicht gemäß Fig. 5 entnehmen.

Wie aus der Schnittdarstellung gemäß Fig. 5 ersichtlich, ist das proximale Ende der Spannzange 20, auf das die Mutter 21 aufgesetzt wird, als Konus 20a ausgebildet. Zur Aufnahme der Spannzange 20 weist die Mutter 21 eine Sacklochbohrung 22 auf, die im Wesentlichen bündig mit der Bohrung 22 abschließend eine Hülse 23 eingesetzt ist, deren proximales Ende einen Gegenkonus 23a zum Konus 20a der Spannzange 20 bildet. Die Einführtiefe der Spannzange 20 in die Hülse 23 und somit auch in die Mutter 21 wird somit dadurch begrenzt, dass der Konus 20a der Spannzange 20 im Wesentlichen formschlüssig am Gegenkonus 23a der Hülse 23 zur Anlage kommt. Um ein Verdrehen der Hülse 23 innerhalb der Mutter 21 beim Aufschrauben der Mutter 21 auf die Spannzange 20 zu verhindern, ist die Hülse 23, beispielsweise durch Verkleben, verdrehsicher in der Sacklochbohrung 22 der Mutter 21 fixiert.

Das Festlegen der Mutter 21 auf der Spannzange 20 erfolgt über ein auf der Spannzange 20 ausgebildetes Außengewinde 20b, das mit einem in der Hülse 23 ausgebildeten Innengewinde 23b zusammenwirkt. Wie aus Fig. 5 ersichtlich, ist an der Hülse 23 distalseitig ein gewindefreier Einstich 23c ausgebildet, um ein verkantungsfreies Aufschrauben der Mutter 21 auf die Spannzange 20 zu erleichtern.

Um durch das Aufschrauben der Mutter 21 auf die Spannzange und durch das Andrücken des Gegenkonus 23a der Hülse 23 auf den Konus 20a der Spannzange 20 eine Fixierung des Zugseils 17 in der Spannzange 20 zu erzielen, ist in der Spannzange mindestens ein in Längsrichtung der Spannzange 20 verlaufender Klemmschlitz 24 ausgebildet, wie dieser in Fig. 4 dargestellt ist. Dieser Klemmschlitz 24 ermöglicht eine Verringerung des Durchmessers der Spannzange 20 und somit ein Verklemmen des in der Spannzange 20 angeordneten Zugseils 17, wenn auf die Spannzange 20 eine radial wirkende Kraftkomponente ausgeübt wird.

Die einwandfreie Betätigung des verschwenkbaren Teils 4a des Arbeitskanals 4 über den Bowdenzug 14 ist abhängig von der richtigen Spannung des Zugseils 17 des Bowdenzugs 14. Zu diesem Zweck ist es notwendig sicherzustellen, dass das Zugseil 17 nicht zu weit oder nicht zu kurz in die Spannzange 20 eingeschoben wird. Wird das Zugseil 17 zu tief in die Spannzange 20 und somit auch in die Mutter 21 eingeschoben, bewirkt dies eine zu straffe Spannung des Zugseils 17, wodurch ein Verstellen des verschwenkbaren Teils 4a kaum noch möglich ist. Ähnlich verhält es sich bei einer zu schwachen Spannung des Zugseils 17 aufgrund einer zu kurzen Einführung des Zugseils 17 in die Spannzange 20.

Zur Montage und Demontage des Spannzange 20 auf dem Zugseil 17 und/oder in der Mutter 21 ist die Spannzange 20 über einen am Steuerungs-Handgriff 15 angeordneten Steuerhebel 26 in Längsrichtung nach vorne und hinten bewegbar.

Bei der in Fig. 5 dargestellten Ausführungsform wird die Einstecktiefe des Zugseils 17 in die Spannzange 20 durch ein hutförmig ausgebildetes Andrückelement 25 begrenzt, das über ein Federelement 27 vorgespannt proximalseitig an der Hülse 23 anliegt. Das Federelement 27 ist dabei so in der Sacklochbohrung 22 der Mutter 12 angeordnet, dass sich das Federelement 27 distalseitig an einem umlaufenden Kragen 25a des hutförmigen Andrückelements 25 und proximalseitig an einer Rückwand der als Hutmutter ausgebildeten Mutter 21 abstützt.

Der Vorteil dieser Konstruktion liegt darin, dass das Zugseil 17 immer in einer fest definierten Position gespannt wird. Dies wird dadurch gewährleistet, dass das Zugseil 17 beim Einführen der Spannzange 20 in die Mutter 21 gegen das Andrückelement 25 drückt, welches seinerseits durch das Federelement 26 immer gegen das Zugseil 17 drückt. Wie aus Fig. 5 ersichtlich, ist eine geschlossene Rückwand 25b des hutförmig ausgebildeten Andrückelements 25 nach außen weisend spitz zulaufend ausgebildet, so dass das im Inneren des Andrückelements 25 angeordnete proximale Ende des Zugseils 17 immer genau mittig zentriert im Andrückelement 25 angeordnet ist und der Druck immer zentriert auf das Zugseil 17 wirkt. Das Zugseil 17 ist so dimensioniert, dass es im Ausgangszustand so lang ist, dass es gegen das Andrückelement 25 andrückt. Durch diese Ausgestaltung des Andrückelements 25 kann die Möglichkeit anwenderbedingter Fehler deutlich minimiert werden.

Um zu gewährleisten, dass das Zugseil 17 bei ausreichender Stabilität mehrmals gespannt werden kann, ist das Zugseil 17 an dem am verschwenkbaren Teil 4a des Arbeitskanals 4 gelagerten distalen Ende hart gelötet.

Die Demontage des Endoskopkopfes 3 erfolgt in umgekehrter Reihenfolge und beginnt mit dem Lösen Mutter 21 zur Freigabe des Zugseils 17. Anschließend wird die Hülse 11 abgeschraubt und der Endoskopkopf 3 vom Endoskopschaft 2 abgezogen.

Ein solchermaßen ausgebildetes Endoskop zeichnet sich dadurch aus, dass eine immer richtige Spannung des Zugseils 17 des Bowdenzugs 14 gewährleistet ist.

### Bezugszeichenliste

- 1: Endoskop
- 2: Endoskopschaft /Schaft
- 3: Endoskopkopf
- 4: Arbeitskanal
- 4a: verschwenkbarer Teil
- 5a: Spülkanal
- 5b: Luftkanal
- 6: Beleuchtungskanal
- 7: Optik
- 8: Führung
- 9: Zuführkanal
- 10: Außengewinde
- 11: Hülse
- 12: Aufnahmeblech
- 13: Schwenkachse
- 14: Bowdenzug
- 15: Steuerungs-Handgriff
- 16: Federelement
- 17: Zugseil
- 18: Bowdenzugmantel
- 19: Bowdenzugkanal
- 20: Spannzange
- 20a: Konus
- 20b: Außengewinde
- 21: Mutter
- 22: Sacklochbohrung
- 23: Hülse
- 23a: Gegenkonus
- 23b: Innengewinde
- 23c: Einstich
- 24: Klemmschlitz
- 25: Andrückelement
- 25a: Kragen
- 25b: Rückwand
- 26: Steuerhebel
- 27: Federelement

## Patentansprüche

1. Endoskop mit einem Schaft (2), in dem mindestens ein Arbeitskanal (4) angeordnet ist und mit einem am distalseitigen Ende des Schaftes (2) angeordneten Endoskopkopf (3), wobei der im Endoskopkopf (3) angeordnete Teil (4a) des Arbeitskanals (4) über einen Bowdenzug (14), der proximalseitig über eine Spannzange (20) an einem Steuerungs-Handgriff (15) gelagert ist, gegenüber dem zugehörigen Arbeitskanal (4) verschwenkbar ist,
**dadurch gekennzeichnet,**
**dass** ein am Steuerungs-Handgriff (15) gelagertes Andrückelement (25) die Einstecktiefe eines Zugseils (17) des Bowdenzugs (14) in die Spannzange (20) begrenzt, wobei an der Spannzange (20) proximalseitig ein Konus (20a) ausgebildet ist, der mit einem Gegenkonus (23a) zusammenwirkt, der in einer auf die Spannzange (20) aufschraubbaren Mutter (21) ausgebildet ist und das Andrückelement (25) über mindestens ein Federelement (27) vorgespannt am Gegenkonus (23a) anliegt, wobei das mindestens eine Federelement (27) in einer Sacklochbohrung (22) der Mutter (21) angeordnet ist.

2. Endoskop nach Anspruch 1, **dadurch gekennzeichnet, dass** im Konus (20a) der Spannzange (20) mindestens ein in Längsrichtung der Spannzange (20) verlaufender Klemmschlitz (24) ausgebildet ist.

3. Endoskop nach Anspruch 1 **dadurch gekennzeichnet, dass** der Gegenkonus (23a) an einer in die Mutter (21) einsetzbaren Hülse (23) ausgebildet ist.

4. Endoskop nach Anspruch 3, **dadurch gekennzeichnet, dass** die Hülse (23), beispielsweise durch Verkleben, verdrehsicher in der Mutter (21) fixierbar ist.

5. Endoskop nach Anspruch 3 oder 4, **dadurch gekennzeichnet, dass** die Hülse (23) distalseitig einen gewindefreien Einstich (23c) aufweist.

6. Endoskop nach einem der Ansprüche 1 bis 5 **dadurch gekennzeichnet, dass** das Andrückelement (25) als hutförmiges Bauteil ausgebildet ist, wobei die axiale Länge des Andrückelements (25) der bevorzugten und maximalen Einstecktiefe des Zugseils in die Spannzange (20) entspricht.

7. Endoskop nach Anspruch 6, **dadurch gekennzeichnet, dass** eine geschlossene Rückwand (25b) des hutförmig ausgebildeten Andrückelements (25) nach außen weisend spitz zulaufend ausgebildet ist.

8. Endoskop nach Anspruch 6 oder 7, **dadurch gekennzeichnet, dass** das Federelement (27) an einem umlaufenden Kragen (25a) des hutförmig ausgebildeten Andrückelements (25) anliegt.

9. Endoskop nach Anspruch 8, **dadurch gekennzeichnet, dass** sich das Federelement (27) distalseitig am Kragen (25a) des Andrückelements (25) und proximalseitig an einer Rückwand der als Hutmutter ausgebildeten Mutter (21) abstützt.

## Claims

1. Endoscope having a shaft (2) in which at least one operating channel (4) is arranged and having an endoscope head (3) arranged on the distal end of the shaft (2), wherein the part (4a) of the operating channel (4) arranged in the endoscope head (3) can be swiveled via a Bowden cable (14), which is mounted via a collet (20) to a control handle (15), opposite the related operating channel (4),
**characterized in that**
a pressing element (25) mounted on the control handle (15) limits the insertion depth of a traction line (17) of the Bowden cable (14) in the collet (20), wherein a cone (20a) is designed on the proximal side on the collet (20) which interacts with a counter-cone (23a) which is designed in a nut (21) which can be screwed onto the collet (20) and the pressing element (25) contacts the counter-cone (23a), pretensioned via at least one spring element (27), wherein the at least one spring element (27) is arranged in a blind hole (22) of the nut (21).

2. Endoscope according to claim 1, **characterized in that** at least one clamping slot (24) is designed in the cone (20a) of the collet (20) running in the longitudinal direction of the collet (20).

3. Endoscope according to claim 1, **characterized in that** the counter-cone (23a) is designed on a sleeve (23) which can be inserted into the nut (21).

4. Endoscope according to claim 3, **characterized in that** the sleeve (23) can be fastened in a rotationally fixed manner in the nut (21), by adhesion, for example.

5. Endoscope according to claim 3 or 4, **characterized in that** the sleeve (23) has a non-threaded insertion (23c) on the distal side.

6. Endoscope according to one of the claims 1 to 5, **characterized in that** the pressing element (25) is designed as a hat-shaped component, wherein the axial length of the pressing element (25) corresponds to the preferred and maximum insertion depth of the traction line in the collet (20).

7. Endoscope according to claim 6, **characterized in that** a closed rear wall (25b) of the hat-shaped pressing element (25) is designed tapering toward the outside.

8. Endoscope according to claim 6 or 7, **characterized in that** the spring element (27) contacts a circumferential collar (25a) of the hat-shaped pressing element (25).

9. Endoscope according to claim 8, **characterized in that** the spring element (27) is supported on the distal side on the collar (25a) of the pressing element (25) and on the proximal side on a rear wall of the nut (21) designed as a cap nut.

## Revendications

1. Endoscope comprenant un corps allongé (2), dans lequel est agencé au moins un canal de travail (4), et comprenant également une tête d'endoscope (3) agencée à l'extrémité distale du corps allongé (2), la partie (4a) du canal de travail (4), qui est agencée dans la tête d'endoscope (3), pouvant pivoter par rapport au canal de travail (4) associé, par l'intermédiaire d'un système de câble sous gaine du type Bowden (14), qui, du côté proximal, est monté, moyennant une pince de serrage (20), sur une poignée de commande (15),
**caractérisé en ce qu'**un élément d'appui (25) monté dans la poignée de commande (15) limite la profondeur d'insertion d'un câble de traction (17) du système de câble sous gaine du type Bowden (14) dans la pince de serrage (20), un cône (20a) étant réalisé du côté proximal sur la pince de serrage (20), ce cône coopérant avec un cône conjugué (23a) réalisé sur un écrou (21) pouvant se visser sur la pince de serrage (20), et l'élément d'appui (25) s'appuyant sous précontrainte contre le cône conjugué (23a) par l'intermédiaire d'au moins un élément de ressort (27), cet élément de ressort (27) étant agencé dans un alésage borgne (22) de l'écrou (21).

2. Endoscope selon la revendication 1, **caractérisé en ce que** dans le cône (20a) de la pince de serrage (20) est formée au moins une fente de serrage (24) s'étendant dans la direction longitudinale de la pince de serrage (20).

3. Endoscope selon la revendication 1, **caractérisé en ce que** le cône conjugué (23a) est formé sur une douille (23) pouvant être insérée dans l'écrou (21).

4. Endoscope selon la revendication 3, **caractérisé en ce que** la douille (23) peut être fixée de manière bloquée en rotation dans l'écrou (21), par exemple par collage.

5. Endoscope selon la revendication 3 ou la revendication 4, **caractérisé en ce que** la douille (23) présente, du côté distal, une encoche (23c) exempte de filetage.

6. Endoscope selon l'une des revendications 1 à 5, **caractérisé en ce que** l'élément d'appui (25) est réalisé en tant que pièce en forme de chapeau, la longueur axiale de l'élément d'appui (25) correspondant à la profondeur d'insertion préférée et maximale du câble de traction dans la pince de serrage (20).

7. Endoscope selon la revendication 6, **caractérisé en ce qu'**une paroi arrière fermée (25b) de l'élément d'appui (25) de configuration en forme de chapeau, est réalisée en forme de pointe dirigée vers l'extérieur.

8. Endoscope selon la revendication 6 ou la revendication 7, **caractérisé en ce que** l'élément de ressort (27) s'appuie sur une collerette périphérique (25a) de l'élément d'appui (25) de configuration en forme de chapeau.

9. Endoscope selon la revendication 8, **caractérisé en ce que** l'élément de ressort (27) s'appuie, côté distal, sur la collerette (25a) de l'élément d'appui (25), et, côté proximal, sur la paroi arrière de l'écrou (21) réalisé sous forme d'écrou borgne.
